# EUROPÄISCHE PATENTANMELDUNG

(11) **EP 1 221 298 A2**
(43) Veröffentlichungstag der Anmeldung: **10.07.2002**
(21) Anmeldenummer: 01130111.6
(22) Anmeldetag: 18.12.2001
(51) Int. Cl.: A61B 5/00

(54) **Vorrichtung und System zum Überwachen von in einem lebenden Körper enthaltenen Objekten**

(30) Priorität: 05.01.2001 DE 10100324
(71) Anmelder: LMB Technologie GmbH, 85445 Schwaig (DE)
(72) Erfinder: Jentsch, Klaus, 85445 Schwaig (DE); Lob, Günter, Prof. Dr., 81377 München (DE)
(74) Vertreter: Winter, Brandl, Fürniss, Hübner, Röss, Kaiser, Polte Partnerschaft

(57) **Zusammenfassung**

Es wird eine Vorrichtung zum Überwachen von in einem lebenden Körper enthaltenen Objekten offenbart, wobei die Vorrichtung aufweist eine Sende/Empfangsvorrichtung, die in den lebenden Körper einbringbar ist und in der das Objekt betreffende Daten speicherbar sind, wobei die das Objekt betreffenden Daten mittels einer sich außerhalb des lebenden Körpers befindenden externen Schreib/Lesevorrichtung berührungslos in die Sende/Empfangsvorrichtung schreibbar und aus dieser lesbar sind, und mindestens eine Sensorvorrichtung aufweist, die mindestens einen zeitlich veränderlichen Zustandsparameter des Objekts überwacht und in Abhängigkeit von einem erfaßten Wert des zeitlich veränderlichen Zustandsparameters einem aktuell vorliegenden Zustand entsprechende Daten zu der Sende/Empfangsvorrichtung überträgt, wobei die dem aktuell vorliegenden Zustand entsprechenden Daten als Reaktion auf das Übertragen von der Sensorvorrichtung in die Sende/Empfangsvorrichtung geschrieben werden. Weiterhin wird ein System zum Überwachen von in einem lebenden Körper enthaltenen Objekten offenbart.

## Beschreibung

Die vorliegende Erfindung betrifft eine Vorrichtung und ein System zum Überwachen von in einem lebenden Körper enthaltenen Objekten.

Auf verschiedenen Gebieten der Medizin ergeben sich bei einem Einbringen von körperfremden Objekten, wie zum Beispiel Implantaten oder Transplantaten oder ähnlichem, verschiedene schwerwiegende Probleme. Zum Beispiel kann es nach einer Transplantation zu Abstoßungserscheinungen hinsichtlich eines transplantierten Organs kommen oder kann sich ein künstliches Implantat, wie zum Beispiel eine Prothese im Laufe der Zeit in ihrer Lage verschieben oder Lockern oder können in beiden Fällen Infektionen auftreten.

Derartige Probleme mußten bisher mit verschiedensten Untersuchungen, wie zum Beispiel Ultraschalluntersuchungen, Röntgenuntersuchungen, usw. frühzeitig erkannt werden, oder ihnen wird, wie dies zum Beispiel bei der Transplantation von Organen der Fall ist, mittels Verabreichung von Medikamenten vorgebeugt.

Derartige Maßnahmen stellen jedoch für betroffene Patienten häufig starke physische und/oder psychische Belastungen dar, wobei jedoch die Maßnahmen als solche unverzichtbar sind.

Es ist demgemäß die Aufgabe der vorliegenden Erfindung, eine Vorrichtung und ein System zum Überwachen von in einem lebenden Körper enthaltenen Objekten zu schaffen, mittels welchen physische und/oder psychische Belastungen betroffener Patienten verringert werden.

Diese Aufgabe wird hinsichtlich der Vorrichtung mit den in Anspruch 1 angegebenen Maßnahmen und hinsichtlich des Systems mit den in Anspruch 12 angegebenen Maßnahmen gelöst.

Genauer gesagt weist eine erfindungsgemäße Vorrichtung zum Überwachen von in einem lebenden Körper enthaltenen Objekten eine Sende/Empfangsvorrichtung, die in den lebenden Körper einbringbar ist und in der das Objekt betreffende Daten speicherbar sind, wobei die das Objekt betreffenden Daten mittels einer sich außerhalb des lebenden Körpers befindenden externen Schreib/Lesevorrichtung berührungslos in die Sende/Empfangsvorrichtung schreibbar und aus dieser lesbar sind; und mindestens eine Sensorvorrichtung auf, die mindestens einen zeitlich veränderlichen Zustandsparameter des Objekts überwacht und in Abhängigkeit von einem erfaßten Wert des zeitlich veränderlichen Zustandsparameters einem aktuell vorliegenden Zustand entsprechende Daten zu der Sende/Empfangsvorrichtung überträgt. Die dem aktuell vorliegenden Zustand entsprechenden Daten werden als Reaktion auf das Übertragen von der Sensorvorrichtung in die Sende/Empfangsvorrichtung geschrieben.

Erfindungsgemäß ist es erkannt worden, daß es zum Überwachen von einem in einem lebenden Körper enthaltenen Objekt vorteilhaft ist, einen zeitlich veränderlichen Zustandsparameter des Objekts zu erfassen und einem aktuell vorliegenden Zustand entsprechende Daten in die Sende/Empfangsvorrichtung zu schreiben.

Ebenso können die das Objekt betreffenden Daten Primärkenndaten des Objekts sein, die durch das Speichern in der Sende/Empfangsvorrichtung an unterschiedlichen Orten für unterschiedliche autorisierte Personen, wie zum Beispiel Ärzte, nutzbar sind.

Durch die zuvor erwähnten Maßnahmen ist es möglich, neben den das Objekt betreffenden Daten dem aktuell vorliegenden Zustand entsprechende Daten verarbeiten zu können, wodurch eine einfache Untersuchungsmöglichkeit von außerhalb des lebenden Körpers mittels der externen Schreib/Lesevorrichtung gegeben ist, ohne daß ein Patient starken Belastungen ausgesetzt wird. Ferner wird durch die Möglichkeit der laufenden Kontrolle des zeitlich veränderlichen Zustandsparameters eine Kontrollmöglichkeit erheblich verbessert.

Weiterhin ist es möglich, frühzeitig das Objekt betreffende negative Entwicklungen zu erkennen, wodurch es ferner möglich ist, ein Qualitätsmanagement von Implantaten und Transplantaten zu schaffen, mittels welchem Folgekosten, wie zum Beispiel durch weitere Operationen, vermieden werden können.

Gemäß einer Ausgestaltung der vorliegenden Erfindung ist das Äußere der Vorrichtung von einem körperverträglichen Material umgeben oder besteht aus diesem.

Gemäß einer weiteren Ausgestaltung der vorliegenden Erfindung ist das Objekt ein Transplantat oder ein Implantat oder ein anderes natürliches oder künstliches Teil oder Ersatzteil.

Gemäß einer weiteren Ausgestaltung der vorliegenden Erfindung sind die dem aktuell vorliegenden Zustand entsprechenden Daten, die in der Sende/Empfangsvorrichtung gespeichert sind, mittels der externen Schreib/Lesevorrichtung lesbar.

Gemäß einer weiteren Ausgestaltung der vorliegenden Erfindung ist die Sende/Empfangsvorrichtung ein Transponder oder ein Bluetooth-Chip.

Gemäß einer weiteren Ausgestaltung der vorliegenden Erfindung erfaßt die Sensorvorrichtung als den zeitlich veränderlichen Zustandsparameter einen Wert einer physischer Funktion, einen Wert einer physiologischen Funktion oder eine Kombination von diesen.

Gemäß einer weiteren Ausgestaltung der vorliegenden Erfindung weist die Vorrichtung weiterhin eine Befestigungsvorrichtung auf, mittels welcher sie innerhalb des lebenden Körpers fixierbar ist.

Gemäß einer weiteren Ausgestaltung der vorliegenden Erfindung werden die dem aktuell vorliegenden Zustand entsprechenden Daten als Reaktion auf das Übertragen von der Sensorvorrichtung direkt in die Sende/Empfangsvorrichtung geschrieben.

Gemäß einer weiteren Ausgestaltung der vorliegenden Erfindung werden die dem aktuell vorliegenden Zustand entsprechenden Daten als Reaktion auf das Übertragen von der Sensorvorrichtung mittels der externen Vorrichtung in die Sende/Empfangsvorrichtung geschrieben.

Ferner kann mit der erfindungsgemäßen Vorrichtung und einer externen Schreib/Lesevorrichtung ein System zum Überwachen von in einem lebenden Körper enthaltenen Objekten mit Merkmalen geschaffen werden, die zu denen bezüglich der zuvor beschriebenen Vorrichtung äquivalent sind.

Die vorliegende Erfindung wird nachstehend anhand von Ausführungsbeispielen unter Bezugnahme auf die beiliegende Zeichnung näher erläutert.

Es zeigt:
- **Fig. 1**: eine schematische Darstellung einer Vorrichtung gemäß einem ersten Ausführungsbeispiel der vorliegenden Erfindung;
- **Fig. 2**: eine schematische Darstellung einer Vorrichtung gemäß einem zweiten Ausführungsbeispiel der vorliegenden Erfindung.

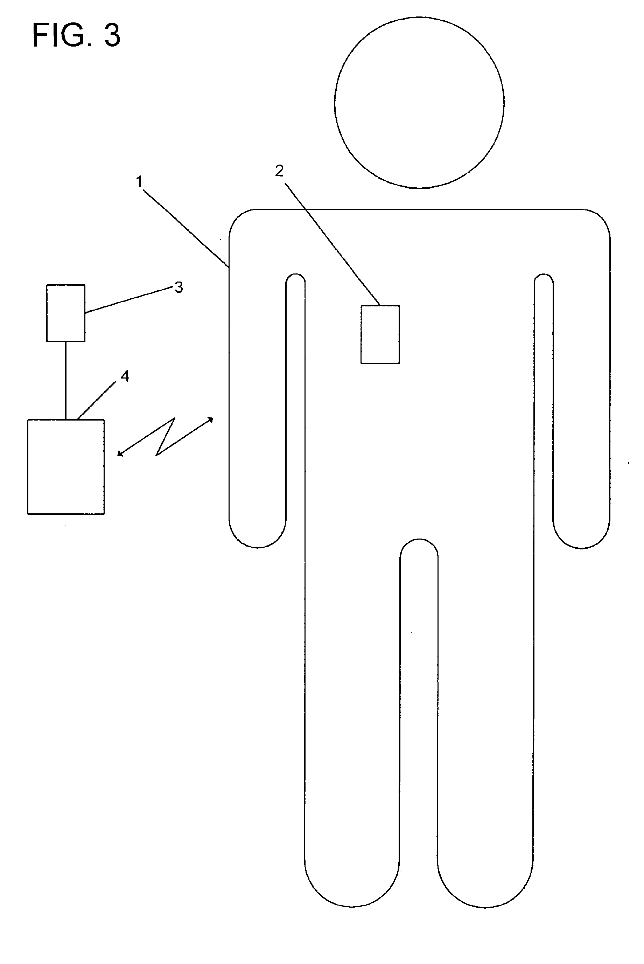

- **Fig. 3**: eine schematische Darstellung einer Vorrichtung gemäß einem dritten Ausführungsbeispiel der vorliegenden Erfindung; und
- **Fig. 4A bis 4E**: eine Anwendung der vorliegenden Erfindung an einem Implantat.

Nachstehend erfolgt die Beschreibung eines ersten Ausführungsbeispiels der vorliegenden Erfindung.

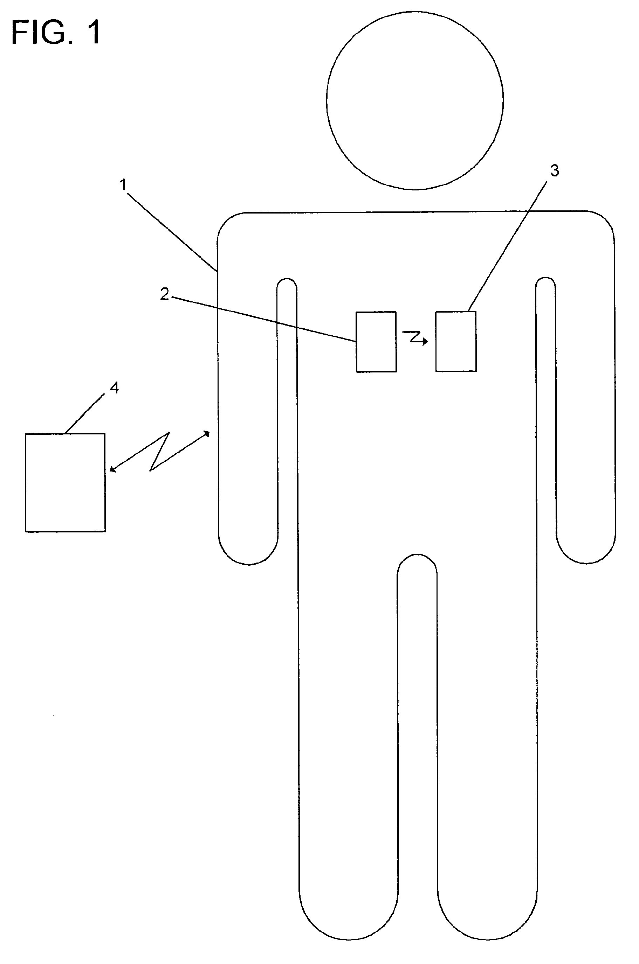

In **Fig. 1** bezeichnet das Bezugszeichen 1 eine symbolische Darstellung eines lebenden Körpers, bezeichnet das Bezugszeichen 2 eine Sende/Empfangsvorrichtung, bezeichnet das Bezugszeichen 3 eine Sensorvorrichtung und bezeichnet das Bezugszeichen 4 eine externe Schreib/Lesevorrichtung.

Im Einsatzfall befindet sich mindestens die Sende/Empfangsvorrichtung 2 innerhalb des lebenden Körpers 1 und befindet sich die externe Schreib/Lesevorrichtung 5 außerhalb des menschlichen Körpers, wie dies durch den lebenden Körper 1 in Fig. 1 angedeutet ist.

Als die Sende/Empfangsvorrichtung 2 kann zum Beispiel ein Transponder oder ein Bluetooth-Chip verwendet werden. Es ist jedoch auch möglich, eine andere Vorrichtung als die Sende/Empfangsvorrichtung 2 zu verwenden, solange die Möglichkeit besteht, daß Daten von außerhalb des lebenden Körpers 1 in die sich innerhalb des lebenden Körpers 1 befindende Sende/Empfangsvorrichtung 2 geschrieben und aus dieser von innerhalb des lebenden Körpers 1 nach außerhalb des lebenden Körpers 1 in die externe Schreib/Lesevorrichtung 4 gelesen werden können.

Abhängig von der Art der verwendeten Sende/Empfangsvorrichtung 2 wird eine geeignete externe Schreib/Lesevorrichtung 4 verwendet. Wenn als die Sende/Empfangsvorrichtung 2 ein Transponder verwendet wird, wird als die externe Schreib/Lesevorrichtung 4 eine externe Transponder-Schreib/Lesevorrichtung verwendet, wenn als die Sende/Empfangsvorrichtung 2 ein Bluetooth-Chip verwendet wird, wird als die externe Schreib/Lese-Vorrichtung 4 eine externe Bluetooth-Schreib/Lesevorrichtung verwendet, usw.

Als die Sensorvorrichtung 3 kann jeder für den vorliegenden Anwendungsfall zweckmäßige Sensor verwendet werden, der imstande ist, einen Wert eines zu überwachenden zeitlich veränderlichen Zustandsparameters eines jeweiligen Objekts zu erfassen. Bei diesem Ausführungsbeispiel der vorliegenden Erfindung werden die von der Sensorvorrichtung 3 erfaßten Werte des zeitlich veränderlichen Zustandsparameters eines zu überwachenden Objekts direkt zu der Sende/Empfangsvorrichtung 2 übertragen und in dieser gespeichert, wie dies anhand des Pfeils zwischen der Sende/Empfangsvorrichtung 2 und der Sensorvorrichtung 3 in **Fig. 1** gezeigt ist. Derartige zu erfassende Zustandsparameter können zum Beispiel jeweilige physiologische oder physische Funktionen innerhalb des lebenden Körpers 1 sein, die sich auch auf Zustände von künstlichen Teilen oder Ersatzteilen beziehen können, wie dies nachstehend detaillierter beschrieben wird. Die jeweils verwendeten Sensorvorrichtungen 3 können sowohl aktive Sensorvorrichtungen, die eine eigene Energieversorgungsquelle benötigen, als auch passive Sensorvorrichtungen sein, die keine eigene Enegerieversorgungsquelle benötigen und zum Beispiel wie ein Transponder mit extern eingekoppelter Fremdenergie arbeiten. Derartige Sensorvorrichtungen 3 zum Erfassen von Werten der zuvor beschriebenen Funktionen sind in der Medizintechnik wohlbekannt und werden hier nicht weiter beschrieben, es sei jedoch angemerkt, daß Sensorvorrichtungen verwendet werden können, die Zustandsparameter von flüssigen Objekten, wie zum Beispiel Blut, oder von gasförmigen Objekten innerhalb des lebenden Körpers erfassen können, wenn dies für den vorliegenden Anwendungsfall erforderlich ist.

Nachstehend erfolgt die Beschreibung des Aufbaus des ersten Ausführungsbeispiels der vorliegenden Erfindung.

Wie es in **Fig. 1** gezeigt ist, befindet sich innerhalb eines lebenden Körpers 1 die Sende/Empfangsvorrichtung 2, die an einer für ein Überwachen eines bestimmten Objekts geeigneten Stelle angeordnet ist. Die Sende/Empfangsvorrichtung 2 besteht aus einem Material, das körperverträglich ist oder ist mit einem derartigen Material umgeben. Derartige Materialien müssen die Fähigkeit aufweisen, daß sie eine Kommunikation von innerhalb des lebenden Körpers 1 nach außerhalb des lebenden Körpers 1 zulassen und beinhalten zum Beispiel Polymerkunststoffe, Polyäthylen, Polyacetat, Polytetrafluoräthylen, Glaskeramik, usw. Die Sende/Empfangsvorrichtung 2 und die Sensorvorrichtung 3 sind bei dem ersten Ausführungsbeispiel der vorliegenden Erfindung beide innerhalb des lebenden Körpers 1 eingebracht und können sowohl in einem einzigen Gehäuse als auch in unterschiedlichen Gehäusen untergebracht sein, solange die gesamte Vorrichtung insgesamt aus einem körperverträglichen Material besteht oder mit diesem bedeckt ist. Ferner weist die gesamte Vorrichtung eine nicht gezeigte Befestigungsvorrichtung auf, mittels welcher die gesamte Vorrichtung innerhalb des menschlichen Körpers 1 fixierbar ist. Eine derartige Befestigungsvorrichtung kann zum Beispiel mittels eines körperverträglichen Klebstoffs realisiert werden, mittels welchem die gesamte Vorrichtung an einem Knochen oder etwas ähnlichem fixierbar ist. Jedoch sind ebenso andere Arten von Befestigungsvorrichtungen möglich.

Ferner ist anzumerken, daß es, obgleich in **Fig. 1** lediglich eine Sende/Empfangsvorrichtung 2 und eine Sensorvorrichtung 3 gezeigt sind, ebenso die Möglichkeit besteht, mehrere andere Sende/Empfangsvorrichtungen 2 und/oder Sensorvorrichtungen 3 zu verwenden, die entweder miteinander integriert oder voneinander getrennt sein können. Schließlich ist die sich außerhalb des lebenden Körpers 1 befindende externe Schreib/Lesevorrichtung 4 vorgesehen, die mit einer nicht gezeigten externen Verarbeitungsvorrichtung, wie zum Beispiel einem Diagnosecomputer, einem Rechner oder ähnlichem drahtlos oder mittels einer zweckmäßigen, zum Beispiel optisch oder elektrisch leitenden Verbindung verbunden werden kann. Die externe Verarbeitungsvorrichtung kann wiederum drahtlos oder mittels einer zweckmäßigen, zum Beispiel optisch oder elektrisch leitenden Verbindung mit einem Computernetzwerk oder dergleichen verbunden werden.

Nachstehend erfolgt die Beschreibung der Funktionsweise des ersten Ausführungsbeispiels der vorliegenden Erfindung.

Wie es bereits erwähnt worden ist, ist die vorliegende Erfindung dazu ausgelegt, ein Objekt innerhalb des lebenden Körpers 1 zu überwachen. Ein derartiges Objekt kann ein transplantiertes Organ, ein künstliches Organ, aber auch jeder andere natürliche oder künstliche Körperersatzteil, wie zum Beispiel eine Prothese oder dergleichen, sein. Je nach zu überwachendem Objekt werden Werte von physiologischen Funktionen und/oder Werte von physischen Funktionen als zeitlich veränderlicher Zustandsparameter mittels geeigneter Sensorvorrichtungen 3 überwacht. Unter physiologischen Funktionen sind hierbei zum Beispiel jeweilige Objekte betreffende chemische und/oder biochemische Reaktionen zu verstehen und unter physischen Funktionen sind hierbei jeweilige Objekte betreffende mechanische Reaktionen zu verstehen.

Als Beispiele derartiger zu überwachender Objekte sind Herz, Niere und andere Organe und alle künstlichen Körperersatzteile, wie zum Beispiel künstliche Organe und Prothesen, zu nennen. Im Fall des Herzens kann zum Beispiel der Herzschlag von der Sensorvorrichtung 3 erfaßt werden und in die Sende/Empfangsvorrichtung 3 übertragen und in dieser gespeichert werden. Im Fall der Prothese oder eines anderen Implantats kann zum Beispiel die Sensorvorrichtung 3 mittels Sende/Empfangsvorrichtugen 2 als Meßpunkte eine Lageänderung der Prothese, wie zum Beispiel ein Schrumpfen der Prothese, erfassen und entsprechende Daten von der Sensorvorrichtung 3 zu der Sende/Empfangsvorrichtung 2 übertragen und in dieser speichern. Diese Anwendung wird an späterer Stelle detaillierter beschrieben. Es sei jedoch angemerkt, daß bei dieser Anwendung die Sensorvorrichtung 3 außerhalb des lebenden Körpers 1 angeordnet ist, wie es ebenso später erläutert wird.

Wenn die erfindungsgemäße Vorrichtung zu verwenden ist, werden bekannte das Organ betreffende oder einen Patienten betreffende Daten mittels der externen Schreib/Lesevorrichtung 5 berührungslos in die Sende/Empfangsvorrichtung geschrieben. Derartige im voraus bekannte Daten können ebenso vor dem Einbringen der Vorrichtung in den lebenden Körper mittels der externen Schreib/Lesevorrichtung 4 in die Sende/Empfangsvorrichtung 3 geschrieben werden. Diese Daten stehen dann bei späteren Überwachungsvorgängen zur Verfügung und können mittels einer jeweiligen externen Schreib/Lesevorrichtung 4 aus der Sende/Empfangsvorrichtung 2 gelesen und einer weiteren Verarbeitung unterzogen werden, können um weitere Daten ergänzt, geändert oder gelöscht werden. Derartige das Objekt betreffende Daten stellen Primärkenndaten oder andere Daten von Transplantaten oder Implantaten dar, die zu jeder beliebigen Zeit an jedem beliebigen Ort berührungslos abrufbar sind. Diese Daten beinhalten zum Beispiel Daten bezüglich einer Operation, wie zum Beispiel Datum, Uhrzeit, Ort, Klinik, verantwortlicher Arzt, nächster Untersuchungstermin usw., Daten bezüglich des Implantats bzw. Transplantats, wie zum Beispiel Hersteller, Seriennumner, Herstellungsdatum, usw. und/oder Daten bezüglich eines Patienten, wie zum Beispiel Einbauhöhe 1 eines Implantats, Einbauhöhe 2 eines Implantats, usw. sowie ggf. weitere Daten, wie zum Beispiel Datum, Uhrzeit, Ort, Spezialarztpraxis, behandelnder Arzt, nächster Untersuchungstermin, Befund usw. Diese Daten werden dazu verwendet um eine Kundendienstund/oder Lebenslaufakte des Transplantats oder Implantats zu erstellen, die leiccht an jedem ort zu jeder Zeit abgerufen werden kann.

Neben diesen das Objekt oder den Patienten betreffenden Daten kann die Sensorvorrichtung 3 zu jedem beliebigen Zeitpunkt und in beliebigen Zeitabständen einen Wert eines aktuell vorliegenden Zustandsparameters erfassen und direkt entweder berührungslos oder mittels einer zweckmäßigen, zum Beispiel elektrisch oder optisch leitenden Verbindung in die Sende/Empfangsvorrichtung 3 schreiben. Ein jeweils erfaßter Wert eines zeitlich veränderlichen Zustandsparameters des Objekts kann dann ebenso zu jedem beliebigen Zeitpunkt ähnlich den zuvor beschriebenen das Objekt betreffenden Daten verwendet und verarbeitet werden.

Die externe Schreib/Lesevorrichtung 4 kann mit einer nicht gezeigten Verarbeitungsvorrichtung, wie zum Beispiel einem Diagnosecomputer, einem an ein Computernetzwerk angeschlossenen Rechner oder ähnlichem entweder drahtlos oder mittels einer zweckmäßigen, zum Beispiel elektrisch oder optisch leitenden Verbindung, verbunden sein, wie es zuvor beschrieben worden ist, so daß über die externe Schreib/Lesevorrichtung 4 die Möglichkeit besteht, sowohl Daten von der externen Verarbeitungsvorrichtung 4 in die Sende/Empfangsvorrichtung 2 zu schreiben als auch von dieser zu lesen, um verschiedene Verarbeitungen durchzuführen.

Mittels der zuvor beschriebenen Vorrichtung besteht die Möglichkeit, eine das Objekt betreffende Diagnose unter Zuhilfenahme von Werten der zeitlich veränderlichen Zustandsparamter zu vereinfachen und besteht ebenso die Möglichkeit, falls es sich um ein künstliches Objekt handelt, die Lebenslaufakte und/oder Kundendienstakte für das zu überwachende Objekt zu erzeugen und in die Sende/Empfangsvorrichtung 2 zu schreiben und aus dieser zu lesen. Allgemein ausgedrückt können ein Objekt betreffende Daten auf die Sende/Empfangsvorrichtung 2 geschrieben, mittels eines externen Rechners oder Computernetzwerks verarbeitet, verändert und/oder ergänzt und wieder in die Sende/Empfangsvorrichtung 2 geschrieben werden.

Die vorliegende Erfindung eignet sich hervorragend für das Überwachen unterschiedlichster Objekte innerhalb eines lebenden Körpers 1.

Nachstehend erfolgt die Beschreibung eines zweiten Ausführungsbeispiels der vorliegenden Erfindung.

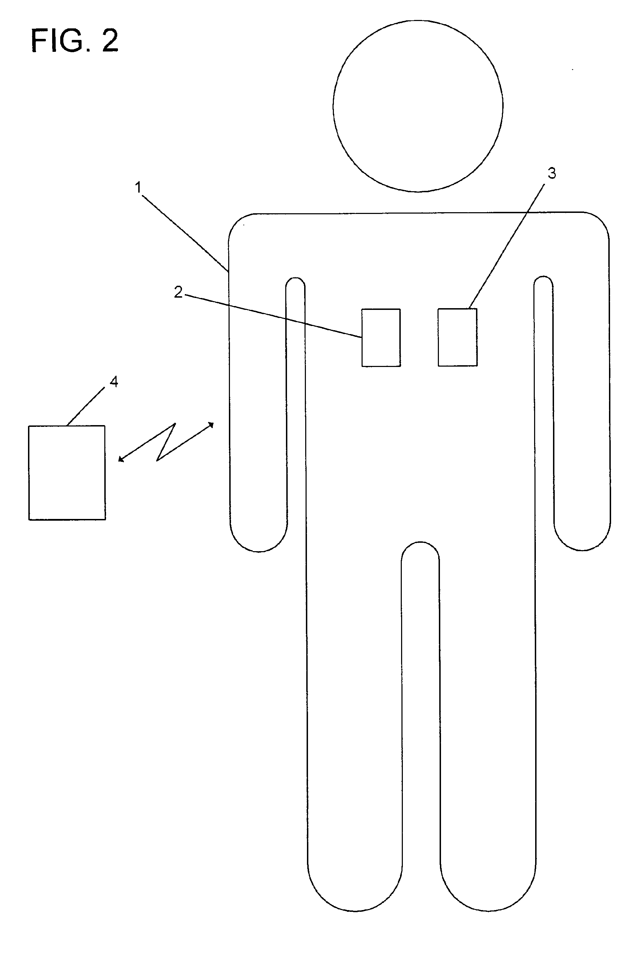

Das zweite Ausführungsbeispiel der vorliegenden Erfindung, wie es in **Fig. 2** gezeigt ist, unterschiedet sich darin von dem ersten Ausführungsbeispiel der vorliegenden Erfindung, daß die Sensorvorrichtung 3 jeweilige Werte der zeitlich veränderlichen Zustandsparameter nicht direkt, sondern indirekt mittels der externen Schreib/Lesevorrichtung 5 entweder berührungslos von der Sensorvorrichtung 3 zu der externen Schreib/Lesevorrichtung 4 überträgt, die dann von dieser in die Sende/Empfangsvorrichtung 2 gespeichert werden. Dadurch können lediglich dann, wenn die externe Schreib/Lesevorrichtung 4 vorhanden ist, jeweilige Daten, die Werten des zeitlich veränderlichen Zustandsparameters eines zu überwachenden Objekts entsprechen, in die Sende/Empfangsvorrichtung 2 geschrieben werden.

Die weitere Funktionsweise des zweiten Ausführungsbeispiels und die weiteren Möglichkeiten zur Verarbeitung der das Objekt betreffenden Daten und der dem Wert des zeitlich veränderlichen Zustands des Objekts betreffenden Daten sind zu der des ersten Ausführungsbeispiels der vorliegenden Erfindung identisch, so daß eine detaillierte Beschreibung an dieser Stelle weggelassen wird.

Nachstehend erfolgt die Beschreibung eines dritten Ausführungsbeispiels der vorliegenden Erfindung.

Das dritte Ausführungsbeispiel der vorliegenden Erfindung unterschiedet sich darin von dem zweiten Ausführungsbeispiel der vorliegenden Erfindung, daß die Sensorvorrichtung 3 außerhalb des lebenden Körpers 1 angeordnet ist. Die Sensorvorrichtung 3 erfaßt die dem zeitlich veränderlichem Zustand betreffenden Daten entweder direkt wie bei dem ersten Ausführungsbeispiel der vorliegenden Erfindung oder indirekt wie bei dem zweiten Ausführungsbeispiel der vorliegenden Erfindung über die externe Schreib/Lesevorrichtung 4 zu der Sende/Empfangsvorrichtung 2, in der diese gespeichert werden.

Die weitere Funktionsweise des dritten Ausführungsbeispiels und die weiteren Möglichkeiten zur Verarbeitung der das Objekt betreffenden Daten und der dem Wert des zeitlich veränderlichen Zustands des Objekts betreffenden Daten sind zu der der ersten und zweiten Ausführungsbeispiele der vorliegenden Erfindung identisch, so daß eine detaillierte Beschreibung an dieser Stelle weggelassen wird.

Nachstehend erfolgt die Beschreibung von Ausgestaltungen der ersten bis dritten Ausführungsbeispiele der vorliegenden Erfindung.

Jede Sende/Empfangsvorrichtung 2 kann eine eindeutige Seriennummer aufweisen. Wenn nunmehr an einem bestimmten Ort, wie zum Beispiel einer Klinik mehrere Patienten mit der erfindungsgemäßen Vorrichtung vorhanden sind, kann mittels der externen Schreib/Lesevorrichtung 4 unter Berücksichtigung einer jeweiligen Seriennummer ausgewählt werden, welche Sende/Empfangsvorrichtung 2 mit der externen Schreib/Lesevorrichtung 4 anzusprechen ist, um Daten in diese zu schreiben oder aus dieser zu lesen, Aufgrund einer derartigen Seriennummer können eine Datenkollision und/oder ein unbeabsichtigtes Ansprechen einer falschen Sende/Empfangsvorrichtung 2 vermieden werden.

Ferner können mittels einer eindeutigen Seriennummer mehrere Sende/Empfangsvorrichtungen 2, die abzufragen sind, mittels eines Zuweisens bestimmter Seriennummern zu einer Gruppe von abzufragenden Sende/Empfangsvorrichtungen 2 gleichzeitig angefragt werden, um Daten in Ihnen zu speichern oder von ihnen zu lesen.

Jede externe Schreib/Lesevorrichtung 4 kann ferner mehrere Kanäle aufweisen, wodurch die Möglichkeit besteht, mehrere Sende/Empfangsvorrichtungen 2 gleichzeitig abzufragen, um unterschiedliche Daten in diese zu schreiben oder von diesen zu lesen.

Die erfindungsgemäße Vorrichtung kann ferner mit anderen Vorrichtungen im Körper gekoppelt sein, die zum Beispiel zum Beispiel eine Medikamentenabgabe im Körper durchführen. Dann erfaßt die Sensorvorrichtung 3 zum Beispiel einen Wert eines zeitlich veränderlichen Parameters, der sich auf die Medikamentenabgabe bezieht. Eine derartige Anwendung wäre zum Beispiel eine implantierte Insulinpumpe, wobei die Sensorvorrichtung 3 dann zum Beispiel entweder eine Dosierung des Insulins oder einen Insulinwert der die Insulinpumpe tragenden Person erfassen kann.

Nachstehend erfolgt die Beschreibung einer Anwendung des vorliegenden Erfindung.

Diese Anwendung bezieht sich auf eine Lageänderung eines Implantats, das vorzugsweise eine Prothese oder ein künstliches Gelenk ist. Diese Anwendung kann jedoch auch bei anderen Implantaten und Transplanaten verwendet werden, wie es leicht ersichtlich ist.

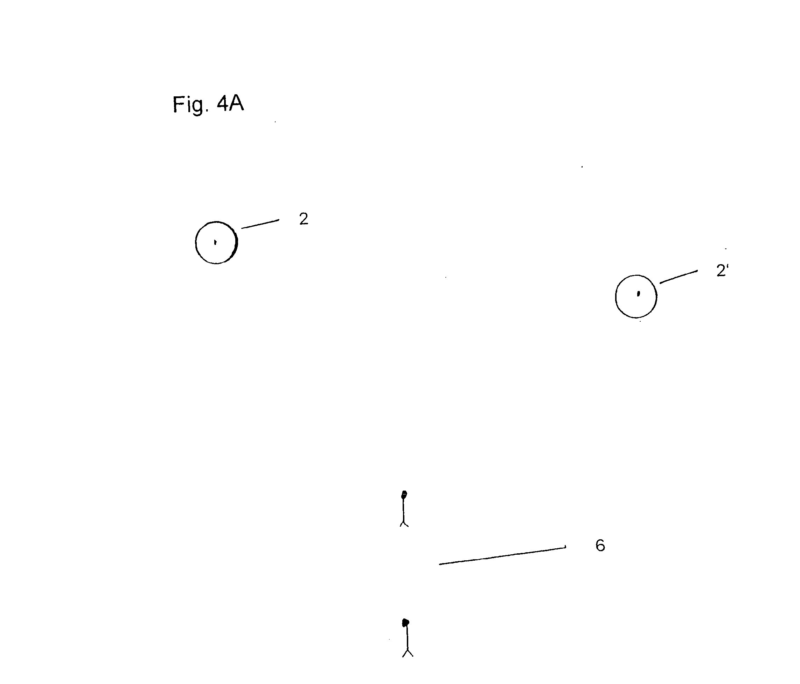

Wie es in **Fig. 4A** gezeigt ist, ist eine erste Sende/Empfangsvorrichung 2 vorgesehen. Diese erste Sende/Empfangsvorrichtung 2' wird als fester Referenzpunkt fest zum Beispiel an einem Knochenstumpf angebracht. Ferner ist eine zweite Sende/Empfangsvorrichtung 2' vorgesehen, die an einer Prothese oder einem künstlichen Gelenk angebracht wird. Ferner weist die Sensorvorrichtung 3 eine vorzugsweise außerhalb des lebenden Körpers 1 angeordnete Mehrfachfensterantenne 6 auf, mittels welcher eine Entfernung zwischen zwei Punkten erfaßt werden kann. Die ist dadurch möglich, daß die Mehrfachfensterantenne 6 mehrere Strahler aufweist, die eine bekannte Entfernung zueinander aufweisen. In diesem Ausführungsbeispiel wird lediglich eine Entfernung im zweidimensionelen Raum zwischen den zwei Sende/Empfangsvorrichtungen 2 und 2' erfaßt, weshalb die Mehrfachfensterantenne 6 lediglich zwei Strahler aufweist. Es ist jedoch auch möglich, diese Anwendung durch Vorsehen weiterer Sende/Empfangsvorrichtungen 2 und einer zweckmäßigen Mehrfachfensterantenne 6 mit einer zweckmäßigen Anzahl von Strahlern derart aufzuweiten, daß Entfernungen und damit Lageänderungen im dreidimensionalen Raum zwischen einer beliebigen Anzahl von Sende/Empfangsvorrichtungen 2 erfaßt werden können.

Um eine Lageänderung der Sende/Empfangsvorrichtung 2' zu erfassen, wird zu einem Zeitpunkt die Entfernung der ersten und zweiten Sende/Empfangsvorrichtungen 2 und 2' zueinander ermittelt.

**Fig. 4A** zeigt eine Anordnung der ersten Sende/Empfangsvorrichtung 2, der zweiten Sende/Empfangsvorrichtung 2' und der Mehrfachfensterantenne 6 mit zwei Strahlern einer bekannten Entfernung.

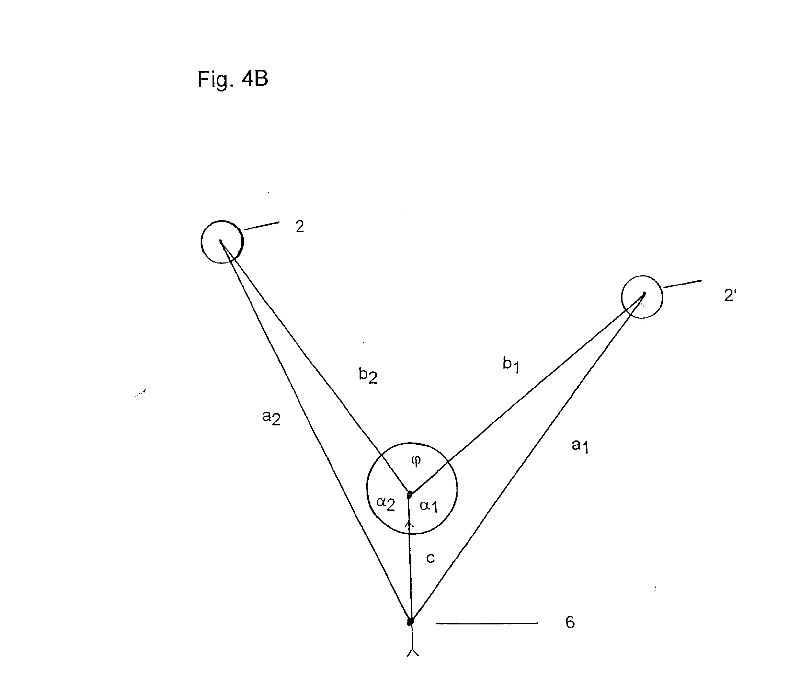

Als erstes werden, wie es in **Fig. 4B** gezeigt ist, die Entfernungen der beiden Sende/Empfangsvorrichtungen 2 und 2' zu der Mehrfachantenne 6 dadurch bestimmt, daß die Mehrfachantenne 6 ein Signal zu den ersten und zweiten Sende- und Empfangsvorrichtungen 2 und 2' überträgt, das dazu führt, daß die Sende/Empfangsvorrichtungen ein Signal zurück zu der Mehrfachantenne übertragen, wodurch mittels der Signallaufzeit die Möglichkeit besteht, die Entfernungen der zwei Sende/Empfangsvorrichtungen 2 und 2' zu den Strahlern der Mehrfachantenne zu ermitteln.

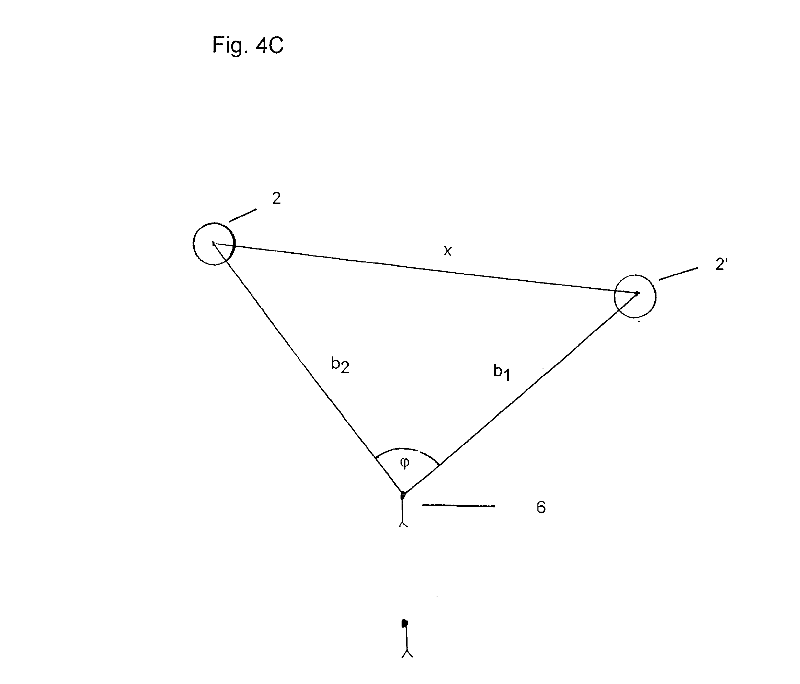

Dadurch besteht ferner die Möglichkeit, anhand zweier ermittelter Entfernungen und rechnerisch mittels Cosinussatz ermittelbaren Winkeln unter erneuter Anwendung des Cosinussatzes die Entfernung zwischen den ersten und zweiten Sende/Empfangsvorrichtungen zu ermitteln, wie dies in **Fig. 4C** gezeigt ist.

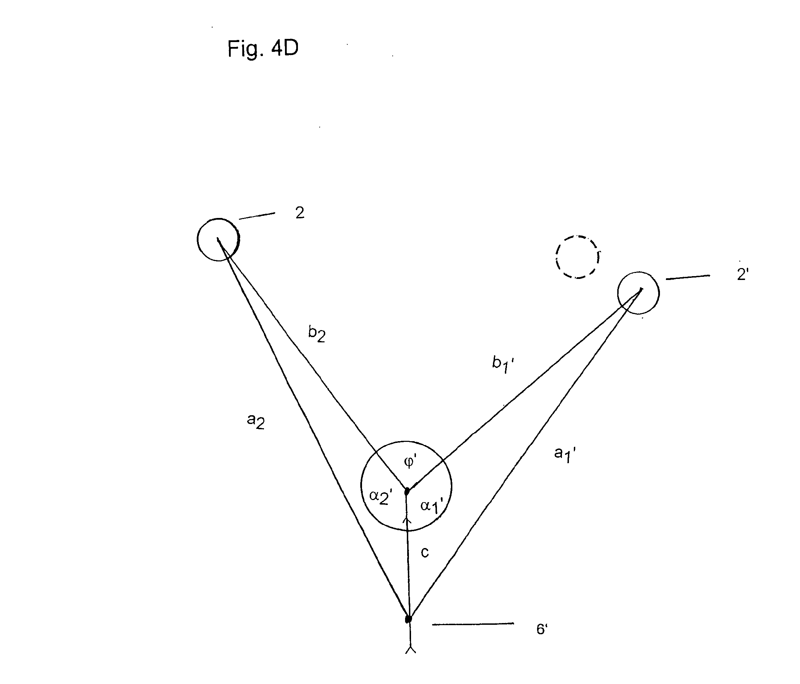

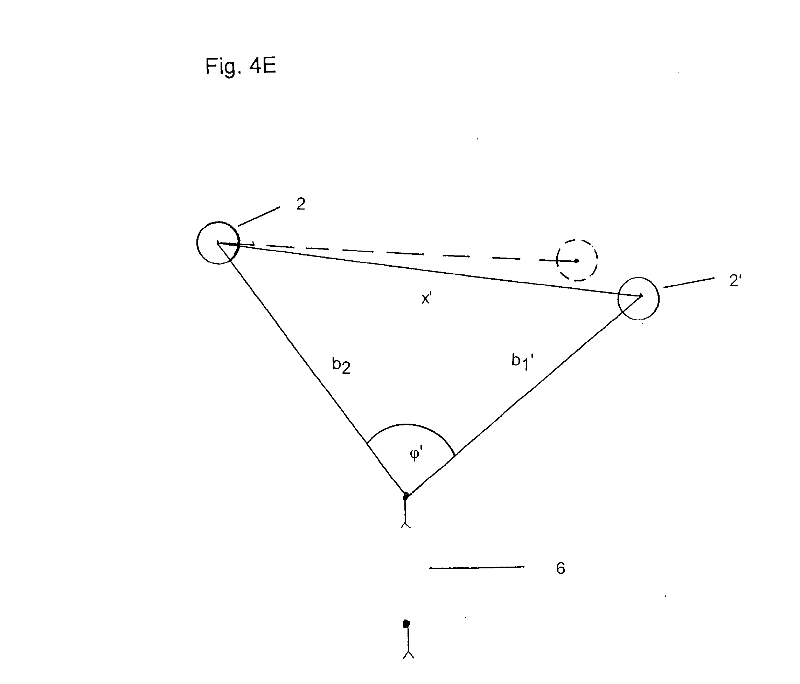

Wenn sich nun, wie es in **Fig. 4D** gezeigt ist, die Lage der zweiten Sende/Empfangsvorrichtung ändert, kann das zuvor erwähnte Verfahren wiederholt werden, wie dies in Fig. **4D** und Fig. **4E** gezeigt ist und kann dann eine Lageänderung anhand der Differenz der ermittelten Entfernungen bestimmt werden.

Mittels dieser Anwendung kann also eine Lageänderung auf einfache Weise erfaßt werden. Daten, die eine jeweils vorliegende Lage der zweiten Sende/Empfangsvorrichtung 2' zu einem Bezugspunkt, das heißt der ersten Sende/Empfangsvorrichtung 2, angeben, werden mittels der Sensorvorrichtung 3 in einer oder beiden der Sende/Empfangsvorrichtungen 2 und 2' oder allgemein einer beliebigen Anzahl jeweils vorhandener Sende/Empfangsvorrichtungen 2 berührungslos gespeichert.

Bezüglich weiterer Merkmale und Vorteile der vorliegenden Erfindung wird ausdrücklich auf die Offenbarung der Zeichnung verwiesen.

## Patentansprüche

1. Vorrichtung zum Überwachen von in einem lebenden Körper enthaltenen Objekten, die aufweist:
mindestens eine Sende/Empfangsvorrichtung, die in den lebenden Körper einbringbar ist und in der das Objekt betreffende Daten speicherbar sind, wobei die das Objekt betreffenden Daten mittels einer sich außerhalb des lebenden Körpers befindenden externen Schreib/Lesevorrichtung berührungslos in die Sende/Empfangsvorrichtung schreibbar und aus dieser lesbar sind; und
mindestens eine Sensorvorrichtung, die mindestens einen zeitlich veränderlichen Zustandsparameter des Objekts überwacht und in Abhängigkeit von einem erfaßten Wert des zeitlich veränderlichen Zustandsparameters einem aktuell vorliegenden Zustand entsprechende Daten zu der Sende/Empfangsvorrichtung überträgt, wobei
die dem aktuell vorliegenden Zustand entsprechenden Daten als Reaktion auf das Übertragen von der Sensorvorrichtung in die Sende/Empfangsvorrichtung geschrieben werden.

2. Vorrichtung nach Anspruch 1, wobei das Äußere der Vorrichtung von einem körperverträglichen Material umgeben ist oder aus diesem besteht.

3. Vorrichtung nach Anspruch 1 oder 2, wobei das Objekt ein Transplantat oder Implantat oder ein anderes natürliches oder künstliches Teil oder Ersatzteil ist.

4. Vorrichtung nach einem der Ansprüche 1 bis 3, wobei die dem aktuell vorliegenden Zustand entsprechenden Daten, die in der Sende/Empfangsvorrichtung gespeichert sind, mittels der externen Schreib/Lesevorrichtung lesbar sind.

5. Vorrichtung nach einem der vorhergehenden Ansprüche, wobei die Sende/Empfangsvorrichtung ein Transponder oder ein Bluetooth-Chip ist.

6. Vorrichtung nach einem der vorhergehenden Ansprüche, wobei die Sensorvorrichtung als den zeitlich veränderlichen Zustandsparameter einen Wert einer physischen Funktion, einen Wert einer physiologischen Funktion oder eine Kombination von diesen erfaßt.

7. Vorrichtung nach einem der vorhergehenden Ansprüche, wobei sie weiterhin eine Befestigungsvorrichtung aufweist, mittels welcher sie innerhalb des lebenden Körpers fixierbar ist.

8. Vorrichtung nach einem der vorhergehenden Ansprüche, wobei die dem aktuell vorliegenden Zustand entsprechenden Daten als Reaktion auf das Übertragen von der Sensorvorrichtung direkt in die Sende/Empfangsvorrichtung geschrieben werden.

9. Vorrichtung nach einem der Ansprüche 1 bis 7, wobei die dem aktuell vorliegenden Zustand entsprechenden Daten als Reaktion auf das Übertragen von der Sensorvorrichtung mittels der externen Vorrichtung in die Sende/Empfangsvorrichtung geschrieben werden.

10. System, das eine Vorrichtung zum Überwachen von in einem lebenden Körper enthaltenen Objekten und eine externe Schreib/Lesevorrichtung aufweist, wobei die Vorrichtung zum Überwachen von in einem lebenden Körper enthaltenen Objekten aufweist:
mindestens eine Sende/Empfangsvorrichtung, die in den lebenden Körper einbringbar ist und in der das Objekt betreffende Daten speicherbar sind, wobei die das Objekt betreffenden Daten mittels der sich außerhalb des lebenden Körpers befindenden externen Schreib/Lesevorrichtung berührungslos in die Sende/Empfangsvorrichtung schreibbar und aus dieser lesbar sind; und
mindestens eine Sensorvorrichtung, die mindestens einen zeitlich veränderlichen Zustandsparameter des Objekts überwacht und in Abhängigkeit von einem erfaßten Wert des zeitlich veränderlichen Zustandsparameters einem aktuell vorliegenden Zustand entsprechende Daten zu der Sende/Empfangsvorrichtung überträgt, wobei
die dem aktuell vorliegenden Zustand entsprechenden Daten als Reaktion auf das Übertragen von der Sensorvorrichtung in die Sende/Empfangsvorrichtung geschrieben werden.

11. System nach Anspruch 10, wobei das Äußere der Vorrichtung zum Überwachen von in einem lebenden Körper enthaltenen Objekten von einem körperverträglichen Material umgeben ist oder aus diesem besteht.

12. System nach Anspruch 10 oder 11, wobei das Objekt ein Tranplantat oder Implantat oder ein anderes natürliches oder künstliches Teil oder Ersatzteil ist.

13. System nach einem der Ansprüche 10 bis 12, wobei die dem aktuell vorliegenden Zustand entsprechenden Daten, die in der Sende/Empfangsvorrichtung gespeichert sind, mittels der externen Schreib/Lesevorrichtung lesbar sind.

14. System nach einem der Ansprüche 10 bis 13, wobei die Sende/Empfangsvorrichtung ein Transponder oder ein Bluetooth-Chip ist.

15. System nach einem der Ansprüche 10 bis 14, wobei die Sensorvorrichtung als den zeitlich veränderlichen Zustandsparameter einen Wert einer physischen Funktion, einen Wert einer physiologischen Funktion oder eine Kombination von diesen erfaßt.

16. System nach einem der Ansprüche 10 bis 15, wobei die Vorrichtung zum Überwachen von in einem lebenden Körper enthaltenen Objekten weiterhin eine Befestigungsvorrichtung aufweist, mittels welcher sie innerhalb des lebenden Körpers fixierbar ist.

17. System nach einem der Ansprüche 10 bis 16, wobei die dem aktuell vorliegenden Zustand entsprechenden Daten als Reaktion auf das Übertragen von der Sensorvorrichtung direkt in die Sende/Empfangsvorrichtung geschrieben werden.

18. Vorrichtung nach einem der Ansprüche 10 bis 16, wobei die dem aktuell vorliegenden Zustand entsprechenden Daten als Reaktion auf das Übertragen von der Sensorvorrichtung mittels der externen Vorrichtung in die Sende/Empfangsvorrichtung geschrieben werden.
